Europäisches Patentamt

European Patent Office

Office européen des brevets

(19)

(11) Publication number: **0 135 347**
**B1**

(12) EUROPEAN PATENT SPECIFICATION

(45) Date of publication of patent specification: **14.03.90**

(51) Int. Cl.⁵: **C 12 N 15/00,** C 12 P 21/00,
C 07 K 13/00 // C12R1/19

(21) Application number: **84305412.3**

(22) Date of filing: **08.08.84**

(54) Human renin and cDNA encoding therefor.

(30) Priority: **12.08.83 JP 147472/83**

(43) Date of publication of application:
**27.03.85 Bulletin 85/13**

(45) Publication of the grant of the patent:
**14.03.90 Bulletin 90/11**

(84) Designated Contracting States:
**BE CH DE FR GB IT LI NL SE**

(56) References cited:
BIOMEDICAL RESEARCH, vol. 3, no. 1, Feb 1982,
Tokyo (JP); T.MASUDA et al.: "Molecular
cloning of DNA complementary to mouse
submandibular gland renin mRNA", pp. 541-545
PROCEDINGS OF THE NATIONAL ACADEMY OF
SCIENCES OF THE USA, vol. 79, no. 19, Aug
1982, Baltimore, MD (US); K.S.MISONO et al.:
"Amino acid sequence of mouse submaxillary
gland renin", pp. 4858-4862

(73) Proprietor: **Murakami, Kazuo**
**116-103 Namiki-2-chome Sakura-mura**
**Niihari-gun Ibaraki Prefecture (JP)**
(73) Proprietor: **Nakanishi, Shigetada**
**10-11 Daigo Ootakacho**
**Fushimi-Ku Kyoto Prefecture (JP)**

(72) Inventor: **Murakami, Kazuo**
**116-103 Namiki-2-chome Sakura-mura**
**Niihari-gun Ibaraki Prefecture (JP)**
Inventor: **Nakanishi, Shigetada**
**10-11 Daigo Ootakacho**
**Fushimi-Ku Kyoto Prefecture (JP)**

(74) Representative: **Perry, Robert Edward et al**
**GILL JENNINGS & EVERY 53-64 Chancery Lane**
**London WC2A 1HN (GB)**

(56) References cited:
NATURE, vol. 298, no. 5869, 01 July 1982, New
York-London; J.-J.PANTHIER et al.: "Complete
amino acid sequence and maturation of the
mouse submaxillary gland renin precursor", pp.
90-92

The file contains technical information
submitted after the application was filed and
not included in this specification

**EP  0 135 347  B1**

(56) References cited:

**PROCEEDINGS OF THE NATIONAL ACADEMY OF SCIENCE OF THE USA, vol. 80, 1983, pp. 7405-7409**

**JOURNAL OF BIOL. CHEMISTRY, vol. 256, 1981, pp. 8164-8171**

**NATURE, vol. 301, 1983, pp. 214-221**
**JOURNAL BIOTECHN., vol. 4, 1986, pp. 205-218**

# EP 0 135 347 B1

## Description

The present invention relates to a human renin and cDNA encoding therefore.

Human renin and its inactive precursor, prorenin, are polypeptides present in the kidney. It has been said that they play an important role in the control of blood pressure. Since they have been isolated from removed internal organs only in very small amounts, their exact amino-acid sequences have not previously been elucidated.

cDNA of human renin and prorenin, based on RNA from removed inernal organs, have now been synthesised, and their base sequences have been identified. This has made it possible to produce the polypeptides using a genetic engineering technique.

The identification and the characterization of two major molecular weight forms of human renin from normal kidney has been disclosed by Slater and Strout Jr. (Reference 13). However the reported aminoacid residue composition is different from that of the present polypeptide sequences.

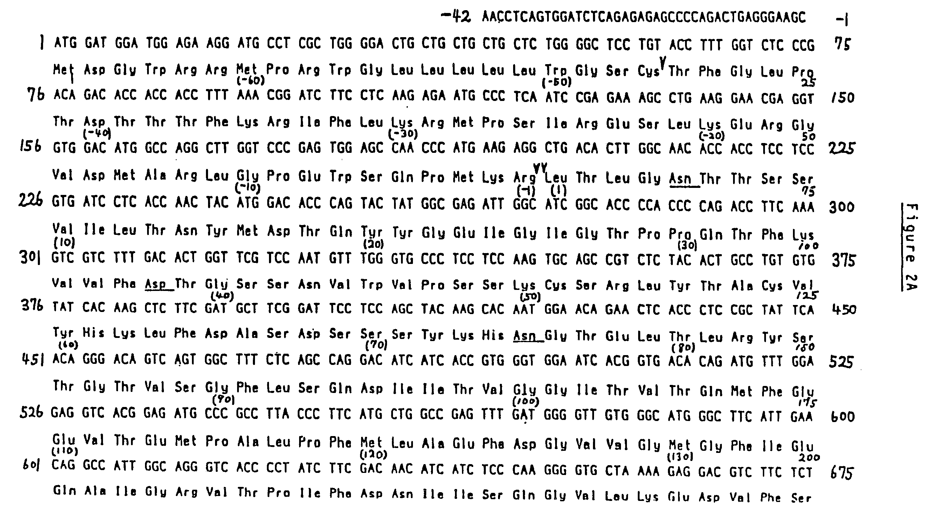

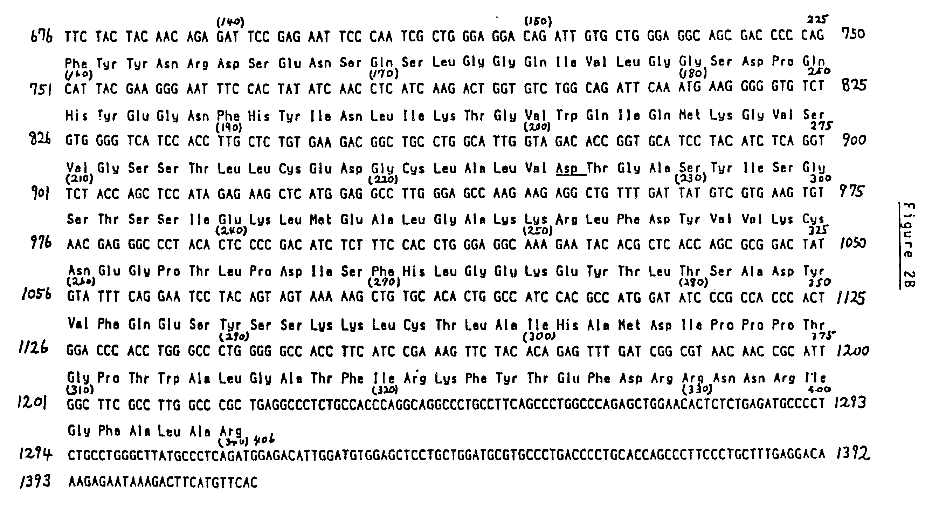

Novel products according to the present invention are a cDNA coding for an amino-acid sequence of a polypeptide portion of human renin; human renin cDNA in a replicatable recombinant DNA sequence; E. coli transformed therewith; and the human renin polypeptide. The human renin cDNA base sequence and the polypeptide encoded thereby are shown in Figures 2A and 2B of the accompanying drawings.

A general procedure for preparing the cDNA is:

Intracytoplasmic RNA is separated from a kidney fragment isolated from a patient showing high serum renin activity, and is treated by using an oligo (dT) cellulose column to obtain mRNA. Cyclic doublestranded DNA is then synthesised from the mRNA according to the method described by Okayama and Berg (Reference 3), i.e. a plasmid joined with cDNA is prepared by annealing a plasmid which has partial deletion in a so-called vector-primer ring and has one terminus of poly (T) to mRNA having one terminus of poly (A); synthesising cDNA by reverse transcription, after treatment of the termini; adding a so-called linker portion to form a ring; and removing the RNA portion from the ring and substituting DNA, using a DNA polymerase.

When transformation of E. coli is effected by using this plasmid, cDNA library of the human kidney is obtained. Since the above-mentioned plasmid is resistant to ampicillin, after screening by utilizing this property, the desired E. coli having the plasmid joined with human renin cDNA is selected by effecting colony hybridization by using cDNA of mouse submandibular gland renin which is assumed to have the amino acid sequence similar to that of human renin.

cDNA is isolated from the resulting strain, its enzyme restriction map is prepared and the base sequence thereof is determined. As the result, a sequence of a non-translated region corresponding to 42 bases followed by a translated region for the peptide corresponding to 1,218 bases and further followed by a non-translated region corresponding to 199 bases has been found.

### Example

#### 1. Isolation of messenger RNA

A kidney fragment (26 g) isolated from a patient suffered from nephrogenous hypertension (serum renin activity was more than 7 times higher than that of a normal person) was homogenized in a solution of 4 M guanidine thiocyanate/0.5 % sodium N-lauroyl sarcosinate/25 mM sodium citrate, pH 7.0/0.1 M 2-mercaptoethanol/0.1 % Antifoam A® (manufactured by Sigma Co.). Intracytoplasmic RNA (65.5 mg) was isolated from the supernatant which was obtained by centrifugation at 8,000 r.p.m. (Kubota, RA—6) for 10 minutes by repeating ethanol precipitation (Reference 1).

The RNA (5.8 mg) was passed through an oligo (dT) cellulose column (Reference 2) to finally obtain poly (A)$^+$ RNA (280 μg).

#### 2. Preparation of cDNA library

Synthesis of cDNA was carried out as follows according to the method described by Okayama and Berg (Reference 3). By the way, as to the vector-primer and the linker described hereinafter, the same materials as those disclosed in Reference 3 were used.

A reverse transcriptase (170 units) was added to a reaction solution containing vector-primer (5.6 μg) and poly (A)$^+$ RNA (22 μg) (50 mM Tris·HCl, pH 8.3/8 mM MgCl$_2$/30 mM KCl/0.3 mM dithiothreitol/2 mM (dATP, dTTP, dGTP, dCTP)/80 μCi [α$^{32}$p] dCTP) and incubated at 37°C for 30 minutes to synthesize the cDNA first strand (mRNA/cDNA-plasmid). This cDNA first strand was reacted with terminal deoxynucleotidyl transferase (30 units) at 37°C for 10 minutes to add dC residue to the 3' terminus and then reacted with Hind III to prepare cDNA having Hind III restriction site and dC residue at both termini. The resulting cDNA (about 2.7 μg) was added to TE buffer (20 μl) (10 mM Tris·HCl, pH 7.41/1 mM EDTA) and then the resulting mixture was mixed with 10X TE buffer (20 μl) (100 mM Tris·HCl, pH 7.4/10 mM EDTA) 1 M NaCl (20 μl), an aqueous solution (36 μl) containing a linker to which dG residue was added (1.62 pmol) and sterilized water (104 μl) and incubated at 65°C for 2 minutes and then at 42°C for 30 minutes to obtain a cyclic plasmid.

The reaction mixture was cooled at 0°C and 100 μl portion thereof was mixed with 10X ligation buffer (100 μl) (200 mM Tris·HCl, pH 7.5/40 mM MgCl$_2$/100 mM ammonium sulfate/ 1 M KCl), 10 mM β-NAD (10 μl), bovine serum albumin (50 μl, 1 mg/ml), sterilized water (738 μl) and an aqueous solution (2 μl) containing E. coli ligase (4 units) and incubated at 12°C overnight.

3

The reaction was mixed with 10 mM (dATP, DTTP, dGTP, dCTP) aqueous solution (4 μl), 10 mM β-NAD (10 μl), an aqueous solution of E. coli DNA ligase (1.5 μl, 2 units/μl), an aqueous solution of RNase H (1.5 μl, 0.74 unit/μl), and an aqueous solution of DNA polymerase I (3 μl, unit/μl), and incubated at 12°C for 1 hour and then at room temperature for one hour to substitute the DNA strand for the RNA portion of the above cyclic plasmid to prepare cyclic double strand DNA (Reference 4). To the reaction mixture (5 μl) was added sterilized water (85 μl) to obtain an aqueous solution of cyclic double strand DNA (90 μl).

E. coli HB101 (accession number: FERM P-7199) was cultivated on LB medium (1 % Bacto-peptone/0.5 % Bacto-yeast extract/0.5 % NaCl/0.1 % glucose). When $A^{650}$ reached to 0.3, cells were collected and then treated with 50 mM $CaCl_2$ solution to form competent cells.

The solution thus treated (100 μl), the above aqueous solution of cyclic double strand DNA (90 μl) and 10 X TE buffer (0.1 M $CaCl_2$/0.3 M $MgCl_2$) (10 μl) were mixed and incubated at 0°C for 20 minutes and then at room temperature for 10 minutes. LB medium (5 ml) was added to the mixture and incubated at 37°C for 1 hour. Cells were collected and suspended in LB medium (80 μl) to obtain a transformant (cDNA library of the human kidney).

3. Screening of renin cDNA carrying cells

The suspension of the transformant thus obtained was inoculated on a nitrocellulose filter placed on LB agar medium (agar 1.5%) containing ampicillin (50 μg/ml) and cultivated at 37°C to allow colonies to grow (5,000—10,000 colonies/filter (diameter 82 mm)). Then, plasmid was amplified by using LB agar medium containing chloramphenicol (500 μg/ml) and plasmide DNA was immobilized on the filter according to the method described by Grunstein and Hogness (Reference 5).

In order to select a strain having a human renin cDNA sequence, colony-hydribization was carried out. That is, mouse renin submandibular gland renin cDNA which had been already cloned (Reference 6) was labeled with $^{32}$P according to nick translation method (Reference 7) to form probe DNA (specific activity: about $1 \times 10^8$ cpm/μg). The above filter on which the plasmid DNA was immobilized was dipped in an aqueous solution of 50 mM Tris·HCl, pH 7.6/10 mM EDTA/1 M NaCl/0.2 % polyvinyl pyrrolidone/0.2 % Ficoll/0.2 % bovine serum albumin/60 μg/ml E. coli DNA/5—10 μg/ml probe DNA and incubated at 55°C for 18 hours. The filter was thoroughly washed at 37°C with an aqueous solution of 0.3 M NaCl/0.03 M sodium citrate/0.1 % sodium lauryl sulfate and subjected to autoradiography to detect spots. With respect to the colonies corresponding to the resulting spots, colony-hybridization was repeated (several tens — several hundreds colonies/filter) to isolate E. coli having renin cDNA.

4. Analysis of human renin cDNA sequence

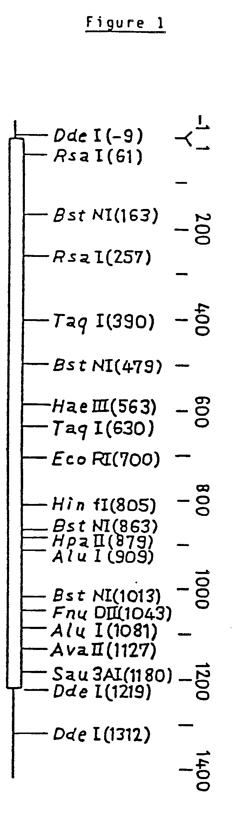

Each of 35 transformant strains which were positive in colony-hybridization was cultivated at 37°C by using LB medium and plasmid DNA was extracted from each strain according to alkali method (Reference 8) to prepare an enzyme restriction map. As the result, cDNA which was joined into each plasmid had a size corresponding to 800—1,600 base pairs (bp) and various enzyme restriction sits were commonly present. The plasmid DNA into which the longest cDNA was joined was designated as pHRn321. The enzyme restriction map of the longest renin cDNA and the entire base sequence thereof determined according to Maxam and Gilbert (Reference 9) were illustrated in Figs. 1 and 2, respectively.

In Figs. 1 and 2, the base sequence is numbered in such a manner that A of ATG encoding for Met at the N terminus is 1 and the symbol (—) is added to the base located in the upper stream side therefrom. In Fig. 1, ☐ shows the region to be translated into the protein and ___ shows the non-translated region. In Fig. 2, the amino acid is numbered in such a manner that Met at the N terminus is 1 and the positions which is deemed to be the restriction site of the signal sequence and that of prorenin are shown by v and vv, respectively. Further, the underlines are provided to Asp in the active centers and the sequence being capable of glycosylation.

This longest renin cDNA except its poly A portion had 1,459 nucleotides. The longest open decoding frame was composed of 1,218 nucleotides encoding for 406 amino acids and had non-translated regions of 42 nucleotides located at the 5′ side and 199 nucleotides located at the 3′ side. (see Fig. 2)

Further, when testing renin mRNA by using this renin cDNA as a probe according to Northern method (Reference 9), it has been found that the number of bases of mRNA is 1,600 nucleotides and the plasmid pHRn321 is that joined with almost entire renin cDNA.

As the result of comparison of the amino acid sequence of the decoded human renin with that of the mouse submandibular gland renin (Reference 11, 12), it has been considered that human renin has 20 hydrophobic signal sequences each of which initiates from Met at the N terminus thereof and human renin protein is resulted from cut between 66th arginine and 67th leucine in the amino acid sequence (see Fig. 2). The molecular weight of the active human renin was 37,200 and the 103rd—107th amino acid sequence (Phe—Asp—Thr—Gly—Ser) and the 291st—294th amino acid sequence (Val—Asp—Thr—Gly) which were deemed to be the active sites were completely identical to the amino acid sequences of the active sites of mouse submandibular gland renin. Further, it showed strong homogeneity with other active sites of acidic protease. The homogenety between the amino acid sequence renin and that of mouse submandibular gland renin was 68 %. Furthermore, the 71st—73rd amino acid sequence (Asn—Thr—Thr) and the 141st—143rd amino acid sequence (Asn—Gly—Thr) are those being capable of addition of a sugar chain and this suggests the possibility that human renin is a glycoprotein.

# EP 0 135 347 B1

Further, due to the difference in the origin of renal cells to be used as the material, there may be partial difference in the base sequence of the resulting cDNA. In addition, it is possible to artificially produce such difference.

References

1. Chirgwin, J. M. *et al., Biochemistry, 18,* 5294 (1979)
2. Aviv, H. *et al., Proc. Natl. Acad. Sci, USA, 69,* 1408 (1972)
3. Okayama, H. *et al., Mol. Cell. Biol. 2,* 161 (1982)
4. Kornberg, A. DNA replication. H. Freeman and Co, San Francisco (1980)
5. Grunstein, M. *et al., Proc, Natl, Acad, Sci. USA, 72,* 3961 (1975)
6. Masuda, T. *et al., Biomedical Res. 3,* 541 (1982)
7. Rigby, P. W. J. *et al., J. Mol. Biol. 113,* 237 (1977)
8. Birnboim, J. C. *et al., Nucleic Acids Res. 7,* 1513 (1979)
9. Maxam, A. M. *et al., Methods Enzymol. 65,* 499, (1980)
10. Alwine, J. C. *et al., Proc. Natl. Acd, Sci. USA, 74,* 5350, (1977)
11. Panthier, J. J. *et al., Nature, 298,* 90, (1982)
12. Misono, K. S. *et al., Proc, Natl, Acad, Sci. USA, 79,* 4858, (1982)
13. Slater, E. E. *et al., The Journ. Biol. Chem, 256,* 8164, (1981)

**Claims**

1. Human renin cDNA, having a base sequence comprising the following base triplets 1 to 406:

ATG GAT GGA TGG AGA AGG ATG CCT CGC TGG

GGA CTG CTG CTG CTG CTC TGG GGC TCC TGT

ACC TTT GGT CTC CCG ACA GAC ACC ACC ACC

TTT AAA CGG ATC TTC CTC AAG AGA ATG CCC

TCA ATC CGA GAA AGC CTG AAG GAA CGA GGT

GTG GAC ATG GCC AGG CTT GGT CCC GAG TGG

AGC CAA CCC ATG AAG AGG CTG ACA CTT GGC

AAC ACC ACC TCC TCC GTG ATC CTC ACC AAC

TAC ATG GAC ACC CAG TAC TAT GGC GAG ATT

GGC ATC GGC ACC CCA CCC CAG ACC TTC AAA

GTC GTC TTT GAC ACT GGT TCG TCC AAT GTT

TGG GTG CCC TCC TCC AAG TGC AGC CGT CTC

TAC ACT GCC TGT GTG TAT CAC AAG CTC TTC

GAT GCT TCG GAT TCC TCC AGC TAC AAG CAC

AAT GGA ACA GAA CTC ACC CTC CGC TAT TCA

ACA GGG ACA GTC AGT GGC TTT CTC AGC CAG

GAC ATC ATC ACC GTG GGT GGA ATG ACG GTG

ACA CAG ATG TTT GGA GAG GTC ACG GAG ATG

```
CCC GCC TTA CCC TTC ATG CTG GCC GAG TTT

GAT GGG GTT GTG GGC ATG GGC TTC ATT GAA

CAG GCC ATT GGC AGG GTC ACC CCT ATC TTC

GAC AAC ATC ATC TCC CAA GGG GTG CTA AAA

GAG GAC GTC TTC TCT TTC TAC TAC AAC AGA

GAT TCC GAG AAT TCC CAA TCG CTG GGA GGA

CAG ATT GTG CTG GGA GGC AGC GAC CCC CAG

CAT TAC GAA GGG AAT TTC CAC TAT ATC AAC

CTC ATC AAG ACT GGT GTC TGG CAG ATT CAA

ATG AAG GGG GTG TCT GTG GGG TCA TCC ACC

TTG CTC TGT GAA GAC GGC TGC CTG GCA TTG

GTA GAC ACC GGT GCA TCC TAC ATC TCA GGT

TCT ACC AGC TCC ATA GAG AAG CTC ATG GAG

GCC TTG GGA GCC AAG AAG AGG CTG TTT GAT

TAT GTC GTG AAG TGT ACC GAG GGC CCT ACA

CTC CCC GAC ATC TCT TTC CAC CTG GGA GGC

AAA GAA TAC ACG CTC ACC AGC GCG GAC TAT

GTA TTT CAG GAA TCC TAC AGT AGT AAA AAG

CTG TGC ACA CTG GCC ATC CAC GCC ATG GAT

ATC CCG CCA CCC ACT GGA CCC ACC TGG GCC

CTG GGG GCC ACC TTC ATC CGA AAG TTC TAC

ACA GAG TTT GAT CGG CGT AAC AAC CGC ATT
                                    406
GGC TTC GCC TTG GCC CGC
```

2. Human renin cDNA, having a base sequence comprising triplets 21 to 406 as defined in claim 1.
3. Human renin cDNA, having a base sequence comprising triplets 67 to 406 as defined in claim 1.
4. Human renin cDNA according to any preceding claim, in a replicatable recombinant DNA sequence.
5. *E. coli* transformed with human renin cDNA according to claim 4.
6. A polypeptide having the following sequence of amino-acides 1 to 406:

Met Asp Gly Trp Arg Arg Met Pro Arg Trp

Gly Leu Leu Leu Leu Leu Trp Gly Ser Cys

Thr Phe Gly Leu Pro Thr Asp Thr Thr Thr

Phe Lys Arg Ile Phe Leu Lys Arg Met Pro

Ser Ile Arg Glu Ser Leu Lys Glu Arg Gly

Val Asp Met Ala Arg Leu Gly Pro Glu Trp

Ser Gln Pro Met Lys Arg Leu Thr Leu Gly

Asn Thr Thr Ser Ser Val Ile Leu Thr Asn

Tyr Met Asp Thr Gln Tyr Tyr Gly Glu Ile

Gly Ile Gly Thr Pro Pro Gln Thr Phe Lys

Val Val Phe Asp Thr Gly Ser Ser Asn Val

Trp Val Pro Ser Ser Lys Cys Ser Arg Leu

Tyr Thr Ala Cys Val Tyr His Lys Leu Phe

Asp Ala Ser Asp Ser Ser Ser Tyr Lys His

Asn Gly Thr Glu Leu Thr Leu Arg Tyr Ser

Thr Gly Thr Val Ser Gly Phe Leu Ser Gln

Asp Ile Ile Thr Val Gly Gly Ile Thr Val

Thr Gln Met Phe Gly Glu Val Thr Glu Met

Pro Ala Leu Pro Phe Met Leu Ala Glu Phe

Asp Gly Val Val Gly Met Gly Phe Ile Glu

Gln Ala Ile Gly Arg Val Thr Pro Ile Phe

Asp Asn Ile Ile Ser Gln Gly Val Leu Lys

Glu Asp Val Phe Ser Phe Tyr Tyr Asn Arg

Asp Ser Glu Asn Ser Gln Ser Leu Gly Gly
Gln Ile Val Leu Gly Gly Ser Asp Pro Gln
His Tyr Glu Gly Asn Phe His Tyr Ile Asn
Leu Ile Lys Thr Gly Val Trp Gln Ile Gln
Met Lys Gly Val Ser Val Gly Ser Ser Thr

```
Leu Leu Cys Glu Asp Gly Cys Leu Ala Leu

Val Asp Thr Gly Ala Ser Tyr Ile Ser Gly

Ser Thr Ser Ser Ile Glu Lys Leu Met Glu

Ala Leu Gly Ala Lys Lys Arg Leu Phe Asp

Tyr Val Val Lys Cys Asn Glu Gly Pro Thr

Leu Pro Asp Ile Ser Phe His Leu Gly Gly

Lys Glu Tyr Thr Leu Thr Ser Ala Asp Tyr

Val Phe Gln Glu Ser Tyr Ser Ser Lys Lys

Leu Cys Thr Leu Ala Ile His Ala Met Asp

Ile Pro Pro Pro Thr Gly Pro Thr Trp Ala

Leu Gly Ala Thr Phe Ile Arg Lys Phe Tyr

Thr Glu Phe Asp Arg Arg Asn Asn Arg Ile
                                    406
Gly Phe Ala Leu Ala Arg
```

7. A polypeptide having an amino-acid sequence comprising amino-acids 21 to 406 as defined in claim 6.

8. A polypeptide having an amino-acid sequence comprising amino-acids 67 to 406 as defined as claim 6.

**Patentansprüche**

1. Menschliches Renin cDNA mit einer Basensequenz, die aus den folgenden Basentripletts 1 bis 406 besteht:

```
1
ATG GAT GGA TGG AGA AGG ATG CCT CGC TGG

GGA CTG CTG CTG CTG CTC TGG GGC TCC TGT
21
ACC TTT GGT CTC CCG ACA GAC ACC ACC ACC

TTT AAA CGG ATC TTC CTC AAG AGA ATG CCC

TCA ATC CGA GAA AGC CTG AAG GAA CGA GGT

GTG GAC ATG GCC AGG CTT GGT CCC GAG TGG
                            67
AGC CAA CCC ATG AAG AGG CTG ACA CTT GGC

AAC ACC ACC TCC TCC GTG ATC CTC ACC AAC

TAC ATG GAC ACC CAG TAC TAT GGC GAG ATT
```

GGC ATC GGC ACC CCA CCC CAG ACC TTC AAA

GTC GTC TTT GAC ACT GGT TCG TCC AAT GTT

TGG GTG CCC TCC TCC AAG TGC AGC CGT CTC

TAC ACT GCC TGT GTG TAT CAC AAG CTC TTC

GAT GCT TCG GAT TCC TCC AGC TAC AAG CAC

AAT GGA ACA GAA CTC ACC CTC CGC TAT TCA

ACA GGG ACA GTC AGT GGC TTT CTC AGC CAG

GAC ATC ATC ACC GTG GGT GGA ATG ACG GTG

ACA CAG ATG TTT GGA GAG GTC ACG GAG ATG

CCC GCC TTA CCC TTC ATG CTG GCC GAG TTT

GAT GGG GTT GTG GGC ATG GGC TTC ATT GAA

CAG GCC ATT GGC AGG GTC ACC CCT ATC TTC

GAC AAC ATC ATC TCC CAA GGG GTG CTA AAA

GAG GAC GTC TTC TCT TTC TAC TAC AAC AGA

GAT TCC GAG AAT TCC CAA TCG CTG GGA GGA

CAG ATT GTG CTG GGA GGC AGC GAC CCC AG

CAT TAC GAA GGG AAT TTC CAC TAT ATC AAC

CTC ATC AAG ACT GGT GTC TGG CAG ATT CAA

ATG AAG GGG GTG TCT GTG GGG TCA TCC ACC

TTG CTC TGT GAA GAC GGC TGC CTG GCA TTG

GTA GAC ACC GGT GCA TCC TAC ATC TCA GGT

TCT ACC AGC TCC ATA GAG AAG CTC ATG GAG

GCC TTG GGA GCC AAG AAG AGG CTG TTT GAT

TAT GTC GTG AAG TGT ACC GAG GGC CCT ACA

CTC CCC GAC ATC TCT TTC CAC CTG GGA GGC

AAA GAA TAC ACG CTC ACC AGC GCG GAC TAT

GTA TTT CAG GAA TCC TAC AGT AGT AAA AAG

CTG TGC ACA CTG GCC ATC CAC GCC ATG GAT

ATC CCG CCA CCC ACT GGA CCC ACC TGG GCC

CTG GGG GCC ACC TTC ATC CGA AAG TTC TAC

ACA GAG TTT GAT CGG CGT AAC AAC CGC ATT

GGC TTC GCC TTG GCC CGC (406)

2. Menschliches Renin cDNA mit einer Basensequenz, die aus den in Anspruch 1 definierten Tripletts 21 bis 406 besteht.

3. Menschliches Renin cDNA mit einer Basensequenz, die aus den in Anspruch 1 definierten Tripletts 67 bis 406 besteht.

4. Menschliches Renin cDNA nach einem der vorhergehenden Ansprüche in einer replikatierbaren rekombinanten DNA-Sequenz.

5. *E. coli* transformiert mit menschlichem Renin cDNA nach Anspruch 4.

6. Ein Polypeptid mit der folgenden Sequenz von Aminosäuren 1 bis 406:

Met (1) Asp Gly Trp Arg Arg Met Pro Arg Trp

Gly Leu Leu Leu Leu Leu Trp Gly Ser Cys

Thr (21) Phe Gly Leu Pro Thr Asp Thr Thr Thr

Phe Lys Arg Ile Phe Leu Lys Arg Met Pro

Ser Ile Arg Glu Ser Leu Lys Glu Arg Gly

Val Asp Met Ala Arg Leu Gly Pro Glu Trp

Ser Gln Pro Met Lys Arg Leu (67) Thr Leu Gly

Asn Thr Thr Ser Ser Val Ile Leu Thr Asn

Tyr Met Asp Thr Gln Tyr Tyr Gly Glu Ile

Gly Ile Gly Thr Pro Pro Gln Thr Phe Lys

Val Val Phe Asp Thr Gly Ser Ser Asn Val

Trp Val Pro Ser Ser Lys Cys Ser Arg Leu

Tyr Thr Ala Cys Val Tyr His Lys Leu Phe

Asp Ala Ser Asp Ser Ser Ser Tyr Lys His

Asn Gly Thr Glu Leu Thr Leu Arg Tyr Ser

Thr Gly Thr Val Ser Gly Phe Leu Ser Gln

Asp Ile Ile Thr Val Gly Gly Ile Thr Val

Thr Gln Met Phe Gly Glu Val Thr Glu Met

```
Pro Ala Leu Pro Phe Met Leu Ala Glu Phe

Asp Gly Val Val Gly Met Gly Phe Ile Glu

Gln Ala Ile Gly Arg Val Thr Pro Ile Phe

Asp Asn Ile Ile Ser Gln Gly Val Leu Lys

Glu Asp Val Phe Ser Phe Tyr Tyr Asn Arg

Asp Ser Glu Asn Ser Gln Ser Leu Gly Gly

Gln Ile Val Leu Gly Gly Ser Asp Pro Gln

His Tyr Glu Gly Asn Phe His Tyr Ile Asn

Leu Ile Lys Thr Gly Val Trp Gln Ile Gln

Met Lys Gly Val Ser Val Gly Ser Ser Thr

    Leu Leu Cys Glu Asp Gly Cys Leu Ala Leu

    Val Asp Thr Gly Ala Ser Tyr Ile Ser Gly

    Ser Thr Ser Ser Ile Glu Lys Leu Met Glu

    Ala Leu Gly Ala Lys Lys Arg Leu Phe Asp

    Tyr Val Val Lys Cys Asn Glu Gly Pro Thr

    Leu Pro Asp Ile Ser Phe His Leu Gly Gly

    Lys Glu Tyr Thr Leu Thr Ser Ala Asp Tyr

    Val Phe Gln Glu Ser Tyr Ser Ser Lys Lys

    Leu Cys Thr Leu Ala Ile His Ala Met Asp

    Ile Pro Pro Pro Thr Gly Pro Thr Trp Ala

    Leu Gly Ala Thr Phe Ile Arg Lys Phe Tyr

    Thr Glu Phe Asp Arg Arg Asn Asn Arg Ile
                                    406
    Gly Phe Ala Leu Ala Arg
```

7. Ein Polypeptid mit einer Aminosäuresequenz, die aus den in Anspruch 6 definierten Aminosäuren 21 bis 406 besteht.

8. Ein Polypeptid mit einer Aminosäurensequenz, die aus den in Anspruch 6 definierten Aminosäuren 67 bis 406 besteht.

**Revendications**

1. ADNc codant al rénine humaine, ayant une séquence de bases comprenant les triplets de bases 1 à 406 suivants:

ATG GAT GGA TGG AGA AGG ATG CCT CGC TGG

GGA CTG CTG CTG CTG CTC TGG GGC TCC TGT

ACC TTT GGT CTC CCG ACA GAC ACC ACC ACC

TTT AAA CGG ATC TTC CTC AAG AGA ATG CCC

TCA ATC CGA GAA AGC CTG AAG GAA CGA GGT

GTG GAC ATG GCC AGG CTT GGT CCC GAG TGG

AGC CAA CCC ATG AAG AGG CTG ACA CTT GGC

AAC ACC ACC TCC TCC GTG ATC CTC ACC AAC

TAC ATG GAC ACC CAG TAC TAT GGC GAG ATT

GGC ATC GGC ACC CCA CCC CAG ACC TTC AAA

GTC GTC TTT GAC ACT GGT TCG TCC AAT GTT

TGG GTG CCC TCC TCC AAG TGC AGC CGT CTC

TAC ACT GCC TGT GTG TAT CAC AAG CTC TTC

GAT GCT TCG GAT TCC TCC AGC TAC AAG CAC

AAT GGA ACA GAA CTC ACC CTC CGC TAT TCA

ACA GGG ACA GTC AGT GGC TTT CTC AGC CAG

GAC ATC ATC ACC GTG GGT GGA ATG ACG GTG

ACA CAG ATG TTT GGA GAG GTC ACG GAG ATG

CCC GCC TTA CCC TTC ATG CTG GCC GAG TTT

GAT GGG GTT GTG GGC ATG GGC TTC ATT GAA

CAG GCC ATT GGC AGG GTC ACC CCT ATC TTC

GAC AAC ATC ATC TCC CAA GGG GTG CTA AAA

GAG GAC GTC TTC TCT TTC TAC TAC AAC AGA

GAT TCC GAG AAT TCC CAA TCG CTG GGA GGA

CAG ATT GTG CTG GGA GGC AGC GAC CCC CAG

12

CAT TAC GAA GGG AAT TTC CAC TAT ATC AAC

CTC ATC AAG ACT GGT GTC TGG CAG ATT CAA

ATG AAG GGG GTG TCT GTG GGG TCA TCC ACC

TTG CTC TGT GAA GAC GGC TGC CTG GCA TTG

GTA GAC ACC GGT GCA TCC TAC ATC TCA GGT

TCT ACC AGC TCC ATA GAG AAG CTC ATG GAG

GCC TTG GGA GCC AAG AAG AGG CTG TTT GAT

TAT GTC GTG AAG TGT ACC GAG GGC CCT ACA

CTC CCC GAC ATC TCT TTC CAC CTG GGA GGC

AAA GAA TAC ACG CTC ACC AGC GCG GAC TAT

GTA TTT CAG GAA TCC TAC AGT AGT AAA AAG

CTG TGC ACA CTG GCC ATC CAC GCC ATG GAT

ATC CCG CCA CCC ACT GGA CCC ACC TGG GCC

CTG GGG GCC ACC TTC ATC CGA AAG TTC TAC

ACA GAG TTT GAT CGG CGT AAC AAC CGC ATT

GGC TTC GCC TTG GCC C̀GC 406

2. ADNc codant pour la rénine humaine, ayant une séquence de bases comprenant les triplets 21 à 406 suivant la revendication 1.

3. ADNc codant pour la rénine humaine, ayant une séquence de bases comprenent les triplets 67 à 406 suivant la revendication 1.

4. ADNc codant pour la rénine humaine suivant l'une quelconque des revendications précédentes, dans une séquence d'ADN recombinant apte à la réplication.

5. *E. coli* transformée avec de l'ADNc codant pour la rénine humaine, suivant la revendication 4.

6. Polypeptide ayant la séquence d'amino-acides 1 à 406 suivante:

Met Asp Gly Trp Arg Arg Met Pro Arg Trp

Gly Leu Leu Leu Leu Trp Gly Ser Cys

Thr Phe Gly Leu Pro Thr Asp Thr Thr Thr

Phe Lys Arg Ile Phe Leu Lys Arg Met Pro

Ser Ile Arg Glu Ser Leu Lys Glu Arg Gly

Val Asp Met Ala Arg Leu Gly Pro Glu Trp

Ser Gln Pro Met Lys Arg Leu Thr Leu Gly

Asn Thr Thr Ser Ser Val Ile Leu Thr Asn

Tyr Met Asp Thr Gln Tyr Tyr Gly Glu Ile

Gly Ile Gly Thr Pro Pro Gln Thr Phe Lys

Val Val Phe Asp Thr Gly Ser Ser Asn Val

Trp Val Pro Ser Ser Lys Cys Ser Arg Leu

Tyr Thr Ala Cys Val Tyr His Lys Leu Phe

Asp Ala Ser Asp Ser Ser Ser Tyr Lys His

Asn Gly Thr Glu Leu Thr Leu Arg Tyr Ser

Thr Gly Thr Val Ser Gly Phe Leu Ser Gln

Asp Ile Ile Thr Val Gly Gly Ile Thr Val

Thr Gln Met Phe Gly Glu Val Thr Glu Met

Pro Ala Leu Pro Phe Met Leu Ala Glu Phe

Asp Gly Val Val Gly Met Gly Phe Ile Glu

Gln Ala Ile Gly Arg Val Thr Pro Ile Phe

Asp Asn Ile Ile Ser Gln Gly Val Leu Lys

Glu Asp Val Phe Ser Phe Tyr Tyr Asn Arg

Asp Ser Glu Asn Ser Gln Ser Leu Gly Gly

Gln Ile Val Leu Gly Gly Ser Asp Pro Gln

His Tyr Glu Gly Asn Phe His Tyr Ile Asn

Leu Ile Lys Thr Gly Val Trp Gln Ile Gln

Met Lys Gly Val Ser Val Gly Ser Ser Thr
Leu Leu Cys Glu Asp Gly Cys Leu Ala Leu

Val Asp Thr Gly Ala Ser Tyr Ile Ser Gly

Ser Thr Ser Ser Ile Glu Lys Leu Met Glu

Ala Leu Gly Ala Lys Lys Arg Leu Phe Asp

Tyr Val Val Lys Cys Asn Glu Gly Pro Thr

Leu Pro Asp Ile Ser Phe His Leu Gly Gly

```
Lys Glu Tyr Thr Leu Thr Ser Ala Asp Tyr

Val Phe Gln Glu Ser Tyr Ser Ser Lys Lys

Leu Cys Thr Leu Ala Ile His Ala Met Asp

Ile Pro Pro Pro Thr Gly Pro Thr Trp Ala

Leu Gly Ala Thr Phe Ile Arg Lys Phe Tyr

Thr Glu Phe Asp Arg Arg Asn Asn Arg Ile
                                    406
Gly Phe Ala Leu Ala Arg
```

7. Polypeptide ayant une séquence d'amino-acides comprenant les amino-acides 21 à 406 suivant la revendication 6.

8. Polypeptides ayant une séquence d'amino-acides comprenent les amino-acides 67 à 406 suivant la revendication 6.

Figure 1

- *Dde* I (-9)
- *Rsa* I (61)

- *Bst* NI (163)

- *Rsa* I (257)

- *Taq* I (390)

- *Bst* NI (479)

- *Hae* III (563)
- *Taq* I (630)

- *Eco* RI (700)

- *Hin* fI (805)
- *Bst* NI (863)
- *Hpa* II (879)
- *Alu* I (909)

- *Bst* NI (1013)
- *Fnu* DII (1043)
- *Alu* I (1081)
- *Ava* II (1127)
- *Sau* 3AI (1180)
- *Dde* I (1219)

- *Dde* I (1312)

1
200
400
600
800
1000
1200
1400

Figure 2A

-42 AACCTCAGTGGATCTCAGAGAGAGCCCCAGACTGAGGGAAGC   -1

1 ATG GAT GGA TGG AGA AGG ATG CCT CGC TGG GGA CTG CTG CTG CTG CTC TGG GGC TCC TGT ACC TTT GGT CTC CCG   75
Met Asp Gly Trp Arg Arg Met Pro Arg Trp Gly Leu Leu Leu Leu Leu Trp Gly Ser Cys Thr Phe Gly Leu Pro
        (-60)                              (-50)                                    25

76 ACA GAC ACC ACC ACC TTT AAA CGG ATC TTC CTC AAG AGA ATG CCC TCA ATC CGA GAA AGC CTG AAG GAA CGA GGT   150
Thr Asp Thr Thr Thr Phe Lys Arg Ile Phe Leu Lys Arg Met Pro Ser Ile Arg Glu Ser Leu Lys Glu Arg Gly
    (-40)                          (-30)                                    (-20)              50

156 GTG GAC ATG GCC AGG CTT GGT CCC GAG TGG AGC CAA CCC ATG AAG AGG CTG ACA CTT GGC AAC ACC ACC TCC TCC   225
Val Asp Met Ala Arg Leu Gly Pro Glu Trp Ser Gln Pro Met Lys Arg Leu Thr Leu Gly Asn Thr Thr Ser Ser
                (-10)                              (-1) (1)                                    75

226 GTG ATC CTC ACC AAC TAC ATG GAC ACC CAG TAC TAT GGC GAG ATT GGC ATC GGC ACC CCA CCC CAG ACC TTC AAA   300
Val Ile Leu Thr Asn Tyr Met Asp Thr Gln Tyr Tyr Gly Glu Ile Gly Ile Gly Thr Pro Pro Gln Thr Phe Lys
(10)                              (20)                              (30)                       100

301 GTC GTC TTT GAC ACT GGT TCG TCC AAT GTT TGG GTG CCC TCC TCC AAG TGC AGC CGT CTC TAC ACT GCC TGT GTG   375
Val Val Phe Asp Thr Gly Ser Ser Asn Val Trp Val Pro Ser Ser Lys Cys Ser Arg Leu Tyr Thr Ala Cys Val
             (40)                              (50)                                          125

376 TAT CAC AAG CTC TTC GAT GCT TCG GAT TCC TCC AGC TAC AAG CAC AAT GGA ACA GAA CTC ACC CTC CGC TAT TCA   450
Tyr His Lys Leu Phe Asp Ala Ser Asp Ser Ser Ser Tyr Lys His Asn Gly Thr Glu Leu Thr Leu Arg Tyr Ser
(60)                              (70)                       Asn      (80)                     150

451 ACA GGG ACA GTC AGT GGC TTT CTC AGC CAG GAC ATC ATC ACC GTG GGT GGA ATC ACG GTG ACA CAG ATG TTT GGA   525
Thr Gly Thr Val Ser Gly Phe Leu Ser Gln Asp Ile Ile Thr Val Gly Gly Ile Thr Val Thr Gln Met Phe Glu
             (90)                              (100)                                          175

526 GAG GTC ACG GAG ATG CCC GCC TTA CCC TTC ATG CTG GCC GAG TTT GAT GGG GTT GTG GGC ATG GGC TTC ATT GAA   600
Glu Val Thr Glu Met Pro Ala Leu Pro Phe Met Leu Ala Glu Phe Asp Gly Val Val Gly Met Gly Phe Ile Glu
(110)                              (120)                              (130)                    200

601 CAG GCC ATT GGC AGG GTC ACC CCT ATC TTC GAC AAC ATC ATC TCC CAA GGG GTG CTA AAA GAG GAC GTC TTC TCT   675
Gln Ala Ile Gly Arg Val Thr Pro Ile Phe Asp Asn Ile Ile Ser Gln Gly Val Leu Lys Glu Asp Val Phe Ser

676 TTC TAC TAC AAC AGA GAT TCC GAG AAT TCC CAA TCG CTG GGA GGA CAG ATT GTG CTG GGA GGC AGC GAC CCC CAG 750 (140) (150) 225
Phe Tyr Tyr Asn Arg Asp Ser Glu Asn Ser Gln Ser Leu Gly Gly Gln Ile Val Leu Gly Gly Ser Asp Pro Gln (160) (170) (180) 250

751 CAT TAC GAA GGG AAT TTC CAC TAT ATC AAC CTC ATC AAG ACT GGT GTC TGG CAG ATT CAA ATG AAG GGG GTG TCT 825
His Tyr Glu Gly Asn Phe His Tyr Ile Asn Leu Ile Lys Thr Gly Val Trp Gln Ile Gln Met Lys Gly Val Ser (190) (200) 275

826 GTG GGG TCA TCC ACC TTG CTC TGT GAA GAC GGC TGC CTG GCA TTG GTA GAC ACC GGT GCA TCC TAC ATC TCA GGT 900
Val Gly Ser Ser Thr Leu Leu Cys Glu Asp Gly Cys Leu Ala Leu Val Asp Thr Gly Ala Ser Tyr Ile Ser Gly (210) (220) (230) 300

901 TCT ACC AGC TCC ATA GAG AAG CTC ATG GAG GCC TTG GGA GCC AAG AAG AGG CTG TTT GAT TAT GTC GTG AAG TGT 975
Ser Thr Ser Ser Ile Glu Lys Leu Met Glu Ala Leu Gly Ala Lys Lys Arg Leu Phe Asp Tyr Val Val Lys Cys (240) (250) 325

976 AAC GAG GGC CCT ACA CTC CCC GAC ATC TCT TTC CAC CTG GGA GGC AAA GAA TAC ACG CTC ACC AGC GCG GAC TAT 1050
Asn Glu Gly Pro Thr Leu Pro Asp Ile Ser Phe His Leu Gly Gly Lys Glu Tyr Thr Leu Thr Ser Ala Asp Tyr (260) (270) (280) 350

1056 GTA TTT CAG GAA TCC TAC AGT AGT AAA AAG CTG TGC ACA CTG GCC ATC CAC GCC ATG GAT ATC CCG CCA CCC ACT 1125
Val Phe Gln Glu Ser Tyr Ser Ser Lys Lys Leu Cys Thr Leu Ala Ile His Ala Met Asp Ile Pro Pro Pro Thr (290) (300) 375

1126 GGA CCC ACC TGG GCC CTG GGG GCC ACC TTC ATC CGA AAG TTC TAC ACA GAG TTT GAT CGG CGT AAC AAC CGC ATT 1200
Gly Pro Thr Trp Ala Leu Gly Ala Thr Phe Ile Arg Lys Phe Tyr Thr Glu Phe Asp Arg Arg Asn Asn Arg Ile (310) (320) (330) 400

1201 GGC TTC GCC TTG GCC CGC TGAGGCCCTCTGCCACCCAGGCAGGCCCTGCCTTCAGCCCTGGCCCAGAGCTGGAACACTCTCTGAGATGCCCCT 1293
Gly Phe Ala Leu Ala Arg 406 (340)

1294 CTGCCTGGGCTTATGCCCTCAGATGGAGACATTGGATGTGGAGCTCCTGCTGGATGCGTGCCCTGACCCCTGCACCAGCCCTTCCCTGCTTTGAGGACA 1392

1393 AAGAGAATAAAGACTTCATGTTCAC

Figure 28

EP 0 135 347 B1